Europäisches Patentamt

European Patent Office

Office européen des brevets

(11) Publication number: **0 555 540 A1**

# EUROPEAN PATENT APPLICATION

(21) Application number: **92120861.7**

(22) Date of filing: **07.12.92**

(51) Int. Cl.5: **C12P 19/12**, //(C12P19/12, C12R1:13),(C12P19/12, C12R1:15),(C12P19/12, C12R1:06)

(30) Priority: **11.12.91 JP 327494/91**
**13.02.92 JP 26841/92**

(43) Date of publication of application:
**18.08.93 Bulletin 93/33**

(84) Designated Contracting States:
**DE FR GB**

(71) Applicant: **AJINOMOTO CO., INC.**
**15-1, Kyobashi 1-chome, Chuo-ku**
**Tokyo 104(JP)**

(72) Inventor: **Tsuchida, Takayasu, c/o Kawasaki Plant**
**Ajinomoto Co., Inc., 1-1 Suzuki-cho, Kawasaki-ku**
**Kawasaki-shi, Kanagawa-ken(JP)**
Inventor: **Murakami, Yutaka, c/o Central Research Lab.**
**Ajinomoto Co., Inc., 1-1 Suzuki-cho, Kawasaki-ku**
**Kawasaki-shi, Kanagawa-ken(JP)**
Inventor: **Nishimoto, Yoshitaka, c/o Kawasaki Plant**
**Ajinomoto Co., Inc., 1-1 Suzuki-cho, Kawasaki-ku**
**Kawasaki-shi, Kanagawa-ken(JP)**
Inventor: **Kotani,Takuya c/o Kawasaki Plant,Ajinomoto Co. Inc**
**1-1 Suzuki-cho,Kawasaki-ku,Kawasaki-shi Kanagawa-ken(JP)**

(74) Representative: **Strehl Schübel-Hopf Groening & Partner**
**Maximilianstrasse 54 Postfach 22 14 55**
**D-80504 München (DE)**

(54) **Method of producing trehalose.**

(57) A method of producing trehalose, in which a microorganism belonging to the genus Brevibacterium, Corynebacterium, Microbacterium or Arthrobacter and having the ability of producing trehalose is cultured in a liquid medium containing sucrose or maltose as an essential carbon source and the trehalose produced and accumulated in the culture is collected therefrom.

In accordance with the method of the present invention, trehalose is produced inexpensively and efficiently by industrial mass-production.

EP 0 555 540 A1

Field of the Invention:

The present invention relates to a method of producing trehalose. Trehalose is expected to be used as a cell activity-retaining agent, a cold-resistant agent and an anti-freezing agent in the field of medicines and foods.

Prior Art:

For producing trehalose, there have heretofore been known a method of extracting trehalose from natural substances such as Selaginella or dry yeast; a method of incubating a microorganism belonging to the genus Arthrobacter with n-alkanes as carbon sources (Agric. Biol. Chem., Vol. 33, pages 190 to 195, 1969), and a method of using a microorganism belonging to the genus Nocardia (Japanese Patent Application Laid-Open No. 50-154485). However, these are not suitable for industrial mass-production of trehalose in view of the cost and the efficiency.

Problems to be Solved by the Invention:

The object of the present invention is to provide a method of producing trehalose inexpensively and efficiently by industrial mass-production.

Means for Solving the Problems:

The present inventors earnestly and repeatedly studied for the purpose of developing a method of producing trehalose inexpensively and efficiently by industrial mass-production and, as a result, have found that by culturing microorganisms belonging to the genus Brevibacterium, Corynebacterium, Microbacterium or Arthrobacter, which have heretofore been known to produce L-glutamic acid and other various amino acids, in a liquid medium containing sucrose or maltose as an essential carbon source, a noticeable amount of trehalose is produced and accumulated in the culture. It was further found, that when the osmotic pressure of the liquid medium is defined to fall within the range of from 1671 to 3974 mmol/kg, the amount of trehalose produced and accumulated noticeably increases. On the basis of the findings, they have completed the present invention.

Specifically, the present invention provides a method of producing trehalose, in which a microorganism belonging to the genus Brevibacterium, Corynebacterium, Microbacterium or Arthrobacter and having the ability of producing trehalose is incubated in a liquid medium containing sucrose or maltose as an essential carbon source and the trehalose produced and accumulated in the culture is collected therefrom. The present invention further provides a method of producing trehalose, in which a microorganisms belonging to the genus Brevibacterium, Corynebacterium, Microbacterium or Arthrobacter and having the ability of producing trehalose is incubated in a liquid medium containing sucrose or maltose as an essential carbon source and having an osmotic pressure (as the osmotic concentration) of from 1671 to 3974 mmol/kg and the trehalose produced and accumulated in the culture is collected therefrom.

The microorganisms to be used in the present invention may be those of any strain belonging to the genus Brevibacterium, Corynebacterium, Microbacterium or Arthrobacter and having the ability of producing trehalose. Specifically, the following strains are suitable examples :
Brevibacterium lactofermentum ATCC13869
Brevibacterium flavum ATCC14067
Brevibacterium divaricatum ATCC21642
Corynebacterium glutamicum ATCC13032
Corynebacterium acetoacidofirum ATCC13870
Corynebacterium lilium ATCC15990
Arthrobacter citoreus ATCC11624
Athrobacter sulfureis ATCC15170
Microbacterium ammoniaphylum ATCC15354
Mutants of these strains to be derived by mutation and having increased trehalose-producing ability may also be used in the present invention.

An important feature of the present invention is that the incubation of the cells of the strains for producing trehalose is effected in a liquid medium containinig sucrose or maltose as an essential carbon source. In general, glucose is often used as a raw material for fermentation of amino acids. In the method of producing trehalose of the present invention, however, the cells of the stated strains are incubated with an essential carbon source of sucrose or maltose whereby a much larger amount of trehalose may be produced than in the case of using glucose as an essential carbon source. Where sucrose is used as an essential carbon source, a substance having a high sucrose content, such as beet molasses may also be used, naturally. By defining the osmotic pressure of the liquid medium to fall within the range of from 1671 to 3974 mmol/kg, the amount of trehalose to be produced and accumulated is noticeably elevated to give a more favorable result.

As components other than the carbon source in the liquid medium, mentioned are nitrogen sources, inorganic salts and organic minor nutrients. As nitrogen sources, for example, usable are ammonium salts, aqueous ammonia, urea as well as corn steep liquor, protein hydrolysates and amino acid mixtures. As inorganic salts, for example, usable are phosphates and magnesium salts. As organic minor nutrients, for example, usable are suitable amounts of thiamine and biotin. Antibiotics such as penicillin and surfactants such as polyoxysorbitan monopalmitate may be added to the medium initially or during the course of incubation, whereby the amount of trehalose to be produced and accumulated may often be elevated.

The way of controlling the osmotic pressure of the liquid medium to fall within the range of from 1671 to 3974 mmol/kg is not specifically limited. For instance, there is mentioned a method of adding a salt such as potassium chloride, sodium chloride, ammonium chloride, potassium sulfate or sodium sulfate, to the medium in an amount of approximately from 0.5 to 7 %.

The incubation is preferably effected under an aerobic condition, the incubating temperature is preferably from 20 to 45°C, and the pH value of the culture is preferably controlled to fall within the range of from 5.0 to 10.0. For adjustment of the pH value, usable are inorganic or organic acidic or alkaline substances as well as urea, calcium carbonate and ammonia gas.

For collecting trehalose from the culture after incubation, any known method, such as ion exchange resin method, resin chromatographic method or ethanol crystallization method, may be employed singly or in combination.

Examples:

Next, the present invention will be explained in more detail by way of the following examples. Determination of trehalose was effected by high performance liquid chromatography (with column of PA-03-S-5 manufactured by YMC).

Example 1:

A liquid medium containing 10 % of the saccharide as shown in Table 1 below as a carbon source and comprising 2 % of ammonium sulfate, 0.1 % of $KH_2PO_4$, 0.1 % of $MgSO_4.7H_2O$, 100 $\mu$g/liter of thiamine hydrochloride, 5 $\mu$g/liter of biotin, 0.036 % (as total nitrogen) of concentrated soybean decomposate liquid, 0.001 % of $FeSO_4.7H_2O$ and 0.001 % of $MnSO_4.4H_2O$ (pH 7.0) was prepared. This was put in plural 500 ml-shaking flasks each in an amount of 20 ml and sterilized under heat at 115°C for 10 minutes. To the medium was added 50 % of calcium carbonate as sterilized under dry heat. Cells of Brevibacterium lactofermentum ATCC13869, Brevibacterium flavum ATCC14067 or Corynebacterium lilium ATCC15990, as previously cultivated on a bouillon-agar slant medium at 30°C for 24 hours, were inoculated to the medium each in an amount of one platinum loop for each flask, and incubation of the cells was effected at 31.5°C in a back-and-forth shaking incubator. The incubation was finished in 48 hours, and the amount of trehalose produced and accumulated in the culture was determined.

Table 1

| Microorganisms Used | Carbon Sources | | | |
| --- | --- | --- | --- | --- |
| | Glucose | Sucrose | Maltose | Beet Molasses |
| B. lactofermentum ATCC13869 | 0.02 | 1.25 | 0.83 | 0.90 |
| B. flavum ATCC14067 | 0.01 | 0.86 | 0.74 | 0.70 |
| C. lilium ATCC15990 | 0.01 | 0.77 | 0.73 | 0.61 |

The numerical value in Table 1 indicates the amount of trehalose accumulated (g/dl) in the culture.

Where beet molasses was used as a carbon source, polyoxysorbitan monopalmitate was added to the medium, at the point when the absorbance of the 1/26 diluted liquid of the culture at 562 nm became 0.6 after initiation of incubation, to have a concentration of 0.4 %, and the incubation was continued further.

As is noted from the results in Table 1 above, incubation of the microorganisms in a medium containing sucrose or maltose yielded a noticeably large amount of trehalose in the culture.

4

Example 2:

A liquid medium containing 10 % of the saccharide as shown in Table 2 below as a carbon source and comprising 2 % of ammonium sulfate, 0.1 % of $KH_2PO_4$, 0.1 % of $MgSO_4.7H_2O$, 100 $\mu$g/liter of thiamine hydrochloride, 100 $\mu$g/liter of biotin, 0.036 % (as total nitrogen ) of concentrated soybean decomposate liquid, 0.001 % of $FeSO_4.7H_2O$ and 0.001 % of $MnSO_4.4H_2O$ (pH 7.0) was prepared. This was put in a plurality of 500 ml-shaking flasks each in an amount of 20 ml and sterilized under heat at 115°C for 10 minutes. To the medium was added 50 % of calcium carbonate as sterilized under dry heat. Cells of Microbacterium ammoniaphylum ATCC15354 or Arthrobacter sulfureis ATCC15170, as previously cultivated on a bouillon-agar slant medium at 30°C for 24 hours, were inoculated to the medium each in an amount of one platinum loop for each flask, and incubation of the cells was effected at 31.5°C in a back-and-forth shaking incubator. Polyoxysorbitan monopalmitate was added to each flask, at the point when the absorbance of the 1/26 diluted liquid of the culture at 562 nm became 0.6 after initiation of incubation, to have a concentration of 0.4 %, and the incubation was continued further. The incubation was finished in 48 hours, and the amount of trehalose as produced and accumulated in the culture was determined. The results are shown in Table 2 below.

Table 2

| Microorganisms Used | Carbon Sources | | | |
|---|---|---|---|---|
| | Glucose | Sucrose | Maltose | Beet Molasses |
| M. ammoniaphylum ATCC15354 | 0.01 | 0.84 | 0.64 | 0.78 |
| A. sulfureis ATCC15170 | 0.01 | 0.80 | 0.72 | 0.81 |

The numerical value in Table 2 indicates the amount of trehalose accumulated (g/dl) in the culture.

As is noted from the results in Table 2 above, incubation of the microorganisms in a medium containing sucrose or maltose yielded a noticeably large amount of trehalose in the culture.

Example 3:

A liquid medium comprising 4 % of glucose, 0.5 % of urea, 0.1 % of $KH_2PO_4$, 0.04 % of $MgSO_4.7H_2O$, 300 $\mu$g/liter of thiamine hydrochloride, 300 $\mu$g/liter of biotin, 0.1 % (as total nitrogens) of concentrated soybean decomposate liquid, 0.001 % of $FeSO_4.7H_2O$ and 0.001 % of $MnSO_4.4H_2O$ (pH 6.5) was prepared. This was put in several 500 ml-shaking flasks each in an amount of 20 ml and sterilized under heat at 110°C for 10 minutes. Cells of microorganisms shown in anyone of Table 3 to Table 8, as previously cultivated on a bouillon-agar slant medium at 31.5°C for 48 hours, were inoculated to the medium each in an amount of one platinum loop for each flask, and incubation of the cells was effected at 31.5°C for 24 hours in a back-and-forth shaking incubator to prepare a seed culture.

The salt as indicated in anyone of Table 3 to Table 8 below was added in the amount also indicated therein to a medium (pH 7.3) comprising 15 % of sucrose, 0.1 % of $KH_2PO_4$, 0.1 % of $MgSO_4.7H_2O$, 300 $\mu$g/liter of thiamine hydrochloride, 300 $\mu$g/liter of biotin, 0.05 % (as total nitrogen) of concentrated soybean decomposate liquid, 0.001 % of $FeSO_4.7H_2O$ and 0.001 % of $MnSO_4.4H_2O$ to prepare a liquid medium. This was put in plural one liter-jar fermenter and sterilized under heat at 120°C for 20 minutes. To each of them was inoculated 15 ml of the previously prepared seed culture, which was then cultivated at 31.5°C with introducing air thereinto at a flow rate of 1/2 vvm and a stirring rate of 700 rpm. The osmotic pressure of the medium at the start of the incubation was measured with 5100C-Vapor Pressure Osmometer (manufactured by Wescor Co.). During the incubation, the medium was controlled to have a pH of 7.3 with ammonia gas. Polyoxysorbitan monopalmitate was added to the medium at the point when the turbidity of the 1/26 diluted liquid of the culture at 562 nm reached 0.60 after the start of the cultivation, to have a concentration of 0.4 %, and the cultivation was continued further. At the point when the sucrose in the culture was consumed completely, the cultivation was terminated.

Table 3

| Microorganisms | Concentration of Potassium Chloride Added (%) | | | | |
|---|---|---|---|---|---|
| | 0 (1545) | 0.5 (1671) | 1.5 (1924) | 3.0 (2304) | 5.0 (2810) |
| B. lactofermentum | | | | | |
| ATCC13869 | 2.2 | 3.1 | 4.0 | 4.3 | 3.2 |
| C. glutamicum | | | | | |
| ATCC13032 | 1.8 | 2.5 | 3.0 | 3.2 | 2.7 |
| A. citoreus | | | | | |
| ATCC11624 | 1.2 | 1.8 | 2.0 | 2.8 | 1.9 |
| M. ammoniaphylum | | | | | |
| ATCC15354 | 1.5 | 2.0 | 2.8 | 2.9 | 2.0 |

The numerical value as parenthesized indicates the osmotic pressure (mmol/kg) of the medium.

The numerical value in the table indicates the amount of trehalose accumulated (g/dl).

EP 0 555 540 A1

Table 4

| Microorganisms | Concentration of Sodium Chloride Added (%) | | | | |
|---|---|---|---|---|---|
| | 0 (1545) | 0.5 (1690) | 1.5 (1835) | 2.0 (2125) | 4.0 (2705) |
| B. divaricatum | | | | | |
| ATCC21642 | 1.6 | 2.3 | 3.0 | 3.2 | 2.3 |
| C. lilium | | | | | |
| ATCC15990 | 1.8 | 2.6 | 3.0 | 3.0 | 2.7 |
| A. citoreus | | | | | |
| ATCC11624 | 1.2 | 1.9 | 2.2 | 2.4 | 2.0 |
| M. ammoniaphylum | | | | | |
| ATCC15354 | 1.5 | 2.4 | 2.8 | 2.9 | 2.4 |

The numerical value as parenthesized indicates the osmotic pressure (mmol/kg) of the medium.

The numerical value in the table indicates the amount of trehalose accumulated (g/liter).

EP 0 555 540 A1

Table 5

| Microorganisms | Concentration of Ammonium Chloride Added (%) | | | | |
|---|---|---|---|---|---|
| | 0 (1545) | 0.5 (1691) | 1.0 (1837) | 2.0 (2129) | 4.0 (2713) |
| B. lactofermentum | | | | | |
| ATCC13869 | 2.2 | 3.0 | 3.9 | 3.9 | 3.1 |
| C. glutamicum | | | | | |
| ATCC13032 | 1.8 | 2.4 | 3.2 | 3.3 | 2.4 |
| A. citoreus | | | | | |
| ATCC11624 | 1.2 | 1.9 | 2.1 | 2.6 | 1.9 |
| M. ammoniaphylum | | | | | |
| ATCC15354 | 1.5 | 2.3 | 2.7 | 2.7 | 2.4 |

The numerical value as parenthesized indicates the osmotic pressure (mmol/kg) of the medium.

The numerical value in the table indicates the amount of trehalose accumulated (g/liter).

EP 0 555 540 A1

Table 6

| Microorganisms | Concentration of Potassium Sulfate Added (%) | | | | |
|---|---|---|---|---|---|
| | 0 (1545) | 0.5 (1711) | 1.0 (2044) | 3.0 (2544) | 7.0 (3876) |
| B. lactofermentum | | | | | |
| ATCC13869 | 2.2 | 2.9 | 3.1 | 3.6 | 3.0 |
| C. glutamicum | | | | | |
| ATCC13032 | 1.8 | 2.4 | 2.8 | 3.2 | 2.9 |
| M. ammoniaphylum | | | | | |
| ATCC15354 | 1.5 | 1.9 | 2.0 | 3.0 | 2.8 |

The numerical value as parenthesized indicates the osmotic pressure (mmol/kg) of the medium.

The numerical value in the table indicates the amount of trehalose accumulated (g/liter).

EP 0 555 540 A1

Table 7

| Microorganisms | Concentration of Sodium Sulfate Added (%) | | | | |
|---|---|---|---|---|---|
| | 0 (1545) | 0.5 (1718) | 1.5 (2065) | 3.0 (2586) | 7.0 (3974) |
| B. lactofermentum | | | | | |
| ATCC13869 | 2.2 | 2.8 | 3.1 | 3.5 | 2.9 |
| C. glutamicum | | | | | |
| ATCC13032 | 1.8 | 2.4 | 2.9 | 3.2 | 2.5 |
| M. ammoniaphylum | | | | | |
| ATCC15354 | 1.5 | 1.9 | 2.1 | 3.0 | 2.0 |

The numerical value as parenthesized indicates the osmotic pressure (mmol/kg) of the medium.

The numerical value in the table indicates the amount of trehalose accumulated (g/liter).

EP 0 555 540 A1

Table 8

| Microorganisms | Concentration of Potassium Chloride/Ammonium Chloride (1/1) Mixture Added (%) | | | | |
|---|---|---|---|---|---|
| | 0 (1545) | 0.5 (1681) | 1.5 (1953) | 2.0 (2089) | 4.0 (2633) |
| B. lactofermentum ATCC13869 | 2.2 | 3.2 | 4.0 | 4.1 | 3.2 |
| C. glutamicum ATCC13032 | 1.8 | 2.6 | 3.0 | 3.8 | 2.7 |
| M. ammoniaphylum ATCC15354 | 1.5 | 2.0 | 2.5 | 3.0 | 2.3 |

The numerical value as parenthesized indicates the osmotic pressure (mmol/kg) of the medium.

The numerical value in the table indicates the amount of trehalose accumulated (g/liter).

From the results of the measurement of the osmotic pressure of the medium and the amount of the trehalose as accumulated in the culture, it is obvious that when the incubation is effected in the medium controlled to have an osmotic pressure falling within the range of from 1671 to 3974 mmol/kg due to addition of a salt thereto, then the amount of the trehalose as produced and accumulated in the medium is noticeably elevated.

Example 4 :

Incubation of Brevibacterium lactofermentum ATCC 13869 was effected in the same manner as in Example 1, except that sucrose was used as the carbon source. One liter of the culture obtained was sterilized, then treated with an anion exchange resin Amberlite IR-4B and a cation exchange resin Amberlite IR-120 and decolored with active charcoal. Next, the decolored liquid was concentrated to about 25 ml, and 75 ml of ethanol was added thereto and left to be cooled to 5°C to obtain 8.5 g of trehalose crystals having a purity of 99 %.

**Claims**

1. A method of producing trehalose, in which a microorganism belonging to the genus Brevibacterium, Corynebacterium, Microbacterium or Arthrobacter and having the ability of producing trehalose is cultured in a liquid medium containing sucrose or maltose as an essential carbon source and the trehalose produced and accumulated in the culture is collected therefrom.

2. The method of producing trehalose according to claim 1, in which the osmotic pressure of the liquid medium, defined as the osmotic concentration, is from 1671 to 3974 mmol/kg.

## DOCUMENTS CONSIDERED TO BE RELEVANT

EP 92120861.7

| Category | Citation of document with indication, where appropriate, of relevant passages | Relevant to claim | CLASSIFICATION OF THE APPLICATION (Int. Cl.5) |
|---|---|---|---|
| P,X | FR - A - 2 671 099 (ORSAN) * Claims * | 1,2 | C 12 P 19/12 //(C 12 P 19/12 C 12 R 1:13) (C 12 P 19/12 C 12 R 1:15) (C 12 P 19/12 C 12 R 1:06) |
| X | CHEMICAL ABSTRACTS, vol. 103, no. 17, October 28, 1985 Columbus, Ohio, USA R.E. LONDONI et al. "Biosynthesis of trehalose by Brevibacterium flavum: use of long range carbon-13-carbon-13 coupling data to characterize triose phosphate isomerase activity", page 397, right column, abstract-no. 138 281u & Biosci. Rep. 1985, 5(6), 509-15 | 1 | |
| A | DE - B - 1 642 708 (KYOWA HOKKO KOGYO) * Claim * | 1 | TECHNICAL FIELDS SEARCHED (Int. Cl.5) C 12 P |

The present search report has been drawn up for all claims

| Place of search | Date of completion of the search | Examiner |
|---|---|---|
| VIENNA | 22-01-1993 | WOLF |

EPO FORM 1503 03.82 (P0401)